# FASCICULE DE BREVET EUROPEEN

(11) **EP 4 099 909 B1**
(45) Date de publication et mention de la délivrance du brevet: **07.08.2024**
(21) Numéro de dépôt: 21708276.7
(22) Date de dépôt: 04.02.2021
(51) Int. Cl.: A61B 8/02, A61B 8/08, A61B 8/00, G01S 15/89

(54) **CEINTURE DE CARDIOTOCOGRAPHIE**
KARDIOTOKOGRAPHIE-GÜRTEL
CARDIOTOCOGRAPHY BELT

(30) Priorité: 04.02.2020 FR 2001080
(43) Date de publication de la demande: 14.12.2022
(73) Titulaire: Nateo Healthcare, 31100 Toulouse (FR)
(72) Inventeur: HARDY, Céline, 31530 MERENVIELLE (FR)
(74) Mandataire: Be IP
(86) Numéro de dépôt international: PCT/FR2021/050211
(87) Numéro de publication internationale: WO 2021/156576

(56) Documents cités:
- EP-A1- 2 213 241
- WO-A1-2004/086085
- WO-A2-2007/120873
- US-A1- 2011 160 591
- US-A1- 2013 331 704
- US-A1- 2020 029 930

## Description

### Domaine technique de l'invention

La présente invention concerne une ceinture de cardiotocographie, abrégée CTG, pour la suite appelée ceinture CTG.

Le mot « CTG » signifie ce qui suit : cardio = coeur, toco = contraction, graphe = écrire.

Durant la grossesse, les battements du coeur de l'enfant sont contrôlés dès la première échographie et sont déjà audibles.

Cette ceinture est utilisée dans le domaine de la gynécologie-obstétrique pour évaluer le bien-être foetal à partir de la 24ème semaine d'aménorrhée pour les grossesses monofoetales et également pour les grossesses gémellaires normales, intermédiaires ou pathologiques.

### Technique antérieure

La ceinture CTG est un instrument composé de capteurs internes et/ou externes mesurant des paramètres physiologiques du foetus et de la mère.

L'enregistrement du rythme cardiaque foetal et des contractions utérines sont deux indicateurs qui permettent d'aider le médecin à faire une interprétation clinique du bien-être foetal au cours de la grossesse comme en cours d'accouchement et en particulier, d'aider à détecter une privation d'oxygène du foetus (asphyxie périnatale).

De manière générale, le capteur ultrasonore est posé sur l'abdomen maternel, et sa position doit être optimisée par le personnel de santé. Une sangle élastique entourant l'abdomen maternel permet le maintien du capteur à une place fixe et permet de garantir un bon couplage.

Toutefois, un mauvais positionnement du capteur ultrasonore entraîne de fausses mesures et par conséquent de mauvais diagnostics.

Un exemple d'art antérieur est le document WO2004/086085 qui décrit une pluralité de capteur ultrasonore pour mesurer le rythme cardiaque foetal. Toutefois, ce document n'est pas satisfaisant car il décrit un transducteur dont le capot est de même dimension (diamètre 8 cm/ 4 cm d'épaisseur) et de même matière plastique (rigide) que les capteurs des CTG du marché. Ce capteur est posé à plat sur l'abdomen de la patiente sans élasticité ni souplesse. Ce transducteur apporte le même niveau d'inconfort et empêche la mobilité de la patiente pendant les mesures comme l'état de l'art des CTG mentionné ci-avant.

Un des objectifs de l'invention est d'assurer le bon positionnement de plusieurs capteurs. Un autre objectif est d'améliorer le confort pour la patiente.

### Présentation de l'invention

La présente invention vise à remédier à ces inconvénients avec une approche totalement novatrice.

A cet effet, la présente invention vise une ceinture de cardiotocographie, remarquable en ce qu'elle a une forme d'une calotte sphérique et comporte au moins dix-neuf transducteurs ultrasonores, appelé TUS, positionnés sur une première couche support en matière élastique, chaque TUS étant positionné dans une cavité support, ladite ceinture comporte une deuxième couche support également en matière élastique qui encapsule lesdits TUS logés dans les cavités supports avec la première couche support, l'assemblage est réalisé par collage de la première couche support avec la deuxième couche support.

Selon un exemple, les cavités support sont reliées entre elles par des liens pour former un réseau régulier de transducteurs.

Grâce à ces dispositions, il y a un positionnement unique au début. Il n'y a pas de repositionnement du capteur par le personnel de santé (sage-femme /obstétricien).

La forme d'une calotte sphérique permet d'être adaptable à toutes les morphologies de femmes enceintes pour toujours rester en contact avec l'abdomen. Cette forme permet de recouvrir une surface hémisphérique.

Un autre avantage est pour la patiente, le fait d'avoir une forme en trois dimensions qui apporte une structure enveloppante et permet la liberté de mouvements comme se mettre debout ou assise sans avoir à repositionner la ceinture. La position de la ceinture est fixe quelle que soit la posture de la patiente (debout, allongée, assise) ou son niveau d'activité.

D'autres avantages sont le confort et la rapidité de l'examen médical (enregistrement CTG).

Le confort est en lien avec le fait que la patiente n'est pas obligée de rester immobile pendant tout l'examen.

La ceinture permet de positionner en une seule fois un réseau de capteurs sur la patiente sans une multitude de potentielles interventions médicales. En effet, le positionnement du capteur unique des CTG du marché (état de l'art) peut être difficile dans les grossesses avant terme en raison de la petite taille du coeur du foetus et parce que le foetus peut se déplacer librement dans l'utérus.

Une fois que le clinicien a déterminé cette position, le capteur est fixé à l'aide d'une sangle élastique passant autour de l'abdomen maternel pour permettre des enregistrements continus du RCF (rythme cardiaque foetal) à un emplacement spécifique du coeur du foetus.

Mais, en raison soit des mouvements du foetus dans l'utérus soit du déplacement du capteur sur l'abdomen de la mère (changement de position de la mère), les enregistrements de RCF peuvent présenter des pertes de signal, les rendant impropres à l'interprétation clinique. En conséquence, la patiente est amenée à attendre le retour du personnel médical pour procéder à un éventuel repositionnement manuel du capteur US et à un nouvel examen.

Dans un autre exemple illustré par les figures ci-après, la ceinture comporte trente-et-un TUS.

Le fait d'avoir une première couche et une deuxième couche rend l'ensemble étanche. Par ce fait, il est nettoyable. On entend par étanche le fait qu'il ne laisse pas passer les liquides.

L'étanchéité est importante pour deux raisons :
- le gel de couplage (gel à base d'eau) qui est déposé par les cliniciens au niveau des TUS ne doit pas pouvoir s'infiltrer à l'intérieur de la partie technique électronique,
- ce gel doit pouvoir être supprimé après examen par simple essuyage et la ceinture doit pouvoir être désinfectée en passant une lingette contenant un produit liquide.

L'invention est avantageusement mise en oeuvre selon les modes de réalisation et les variantes exposées ci-après, lesquelles sont à considérer individuellement ou selon toute combinaison techniquement opérante.

Dans un mode de réalisation, lesdites cavités supports étant disposées de la manière suivante dont au moins :
- cinq cavités supports suivent une ligne centrale définissant une ligne de symétrie et séparées chacune par un lien rigide,
- quatre cavités supports suivent une première ligne parallèle à la ligne centrale, la première ligne est proximale à la ligne centrale et située d'un côté, chaque cavité support de la première ligne est positionnée en alternance avec deux cavités supports de la ligne centrale et reliée à chacune des deux cavités supports de la ligne centrale par un lien rigide,
- quatre cavités supports suivent une deuxième ligne parallèle à la ligne centrale, la deuxième ligne est proximale à la ligne centrale et située de l'autre côté de la ligne centrale, chaque cavité support de la deuxième ligne est positionnée en alternance avec deux cavités supports de la ligne centrale et reliée à chacune des deux cavités supports de la ligne centrale par un lien rigide,
- trois cavités supports suivent une troisième ligne parallèle à la ligne centrale, la troisième ligne est distale à la ligne centrale et située du côté de la première ligne, chaque cavité support de la troisième ligne est positionnée en alternance avec deux cavités supports de la première ligne et reliée à chacune des deux cavités supports de la première ligne par un lien rigide,
- trois cavités supports suivent une quatrième ligne parallèle à la ligne centrale, la quatrième ligne est distale à la ligne centrale et située du côté de la deuxième ligne, chaque cavité support de la quatrième ligne est positionnée en alternance avec deux cavités supports de la deuxième ligne et reliée à chacune des deux cavités supports de la deuxième ligne par un lien rigide.

On entend par rigide le fait d'avoir un lien entre deux cavités support, ce lien est selon un exemple en matière thermoplastique qui admet une certaine flexibilité.

Dans un mode de réalisation, l'épaisseur de la première couche ou de la deuxième couche support est comprise entre 1 et 2 mm, de préférence compris entre 1,3 et 1,7 mm.

Dans un mode de réalisation, la distance proximale entre la première ligne et la ligne centrale ou la deuxième ligne et la ligne centrale est compris entre 32 et 40 mm. Les distances doivent être comprises comme les écartements entre les entraxes des transducteurs.

L'espacement entre capteur ne doit pas être supérieur à 45 mm (avec une tolérance de 1 mm) car cela générerait des aires cardiaques potentiellement non couvertes par les champs ultrasonores.

Dans un mode de réalisation, la distance distale entre la troisième ligne et la ligne centrale ou la quatrième ligne et la ligne centrale est comprise entre 64 et 80 mm.

Dans un mode de réalisation, le lien rigide entre la ligne centrale et la première ligne ou la ligne centrale et la deuxième ligne forme un angle compris entre 50 et 70° par rapport à la ligne centrale.

Dans un mode de réalisation, le lien rigide entre la première ligne et la troisième ligne ou la deuxième ligne et la quatrième ligne forme un angle compris entre 50 et 70° par rapport à la ligne centrale.

Dans un mode de réalisation, chaque lien rigide a une longueur entre 38 et 46 mm.

Dans un mode de réalisation, la matière élastique est du silicone.

Dans un mode de réalisation, un trou débouchant (de repérage) est situé entre la première ligne et la ligne centrale, ledit trou débouchant est sensiblement en position centrale de ladite ceinture. L'effet technique est que ce trou débouchant facilite la pose par l'utilisateur de la ceinture. Lors de la pose de la ceinture, l'utilisateur ou le praticien utilise le trou débouchant comme un trou de repérage pour localiser le nombril de la patiente. Lorsque la ceinture comporte la deuxième couche support, le trou débouchant est étanche et ne laisse pas passer les liquides entre la première couche support et la deuxième couche support.

Dans un mode de réalisation, le trou débouchant est sensiblement en position centrale de ladite ceinture. Dans cette variante, le trou est indépendant de la position des lignes définies ci-avant. Toutefois les effets techniques associés reste inchangés. Avantageusement le trou débouchant (de repérage) est situé dans une zone de la ceinture qui correspond à une position typique des nombrils des patientes, lorsque la ceinture est correctement positionnée. Ainsi le trou de repérage peut indiquer que la ceinture est correctement positionnée sur la patiente. Avantageusement le trou de repérage a un diamètre légèrement supérieur à un index humain, soit 10 à 12mm.

Le trou de repérage est indépendant du nombre de la forme ou de la position des transducteurs.

### Brève description des figures

D'autres avantages, buts et caractéristiques de la présente invention ressortent de la description qui suit faite, dans un but explicatif et nullement limitatif, en regard des dessins annexés, dans lesquels :
[Fig. 1] la figure 1 représente une vue en perspective d'une partie de ceinture de cardiotocographie, objet de la présente invention ;
[Fig. 2] la figure 2 représente les éléments de la figure 1 recouverts par une membrane composée de la première couche support et de la deuxième couche support ;
[Fig. 3] la figure 3 représente un zoom d'une partie de la figure 1 pour montrer un angle selon l'exemple de réalisation de la figure 1 ;
[Fig. 4] la figure 4 représente une vue en coupe d'une ceinture de cardiotocographie, montrant la première couche support et la deuxième couche support, le trou de repérage et les TUS ;
[Fig. 5] la figure 5 représente une autre vue en coupe d'une ceinture de cardiotocographie montrant le collage de la première couche support avec la deuxième couche support ;
[Fig. 6] La figure 6 représente une autre vue en coupe selon la ligne LC ;
[Fig. 7] La figure 7 représente une vue de détail de la coupe précédente.

### Description des modes de réalisation

La ceinture a une forme de courbe, hémisphérique ou une calotte sphérique pour suivre les courbes du ventre d'une femme enceinte.

Dans un mode de réalisation, la ceinture comporte sur ses côtés des attaches pour une bande élastique qui passe derrière le dos de la femme enceinte.

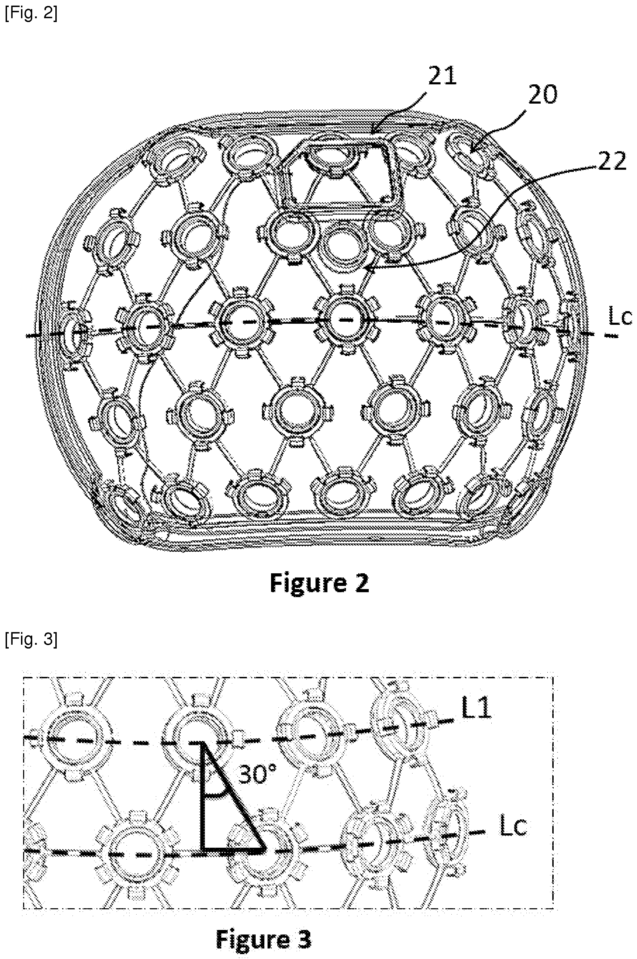

Dans un mode de réalisation, la calotte sphérique ne comporte qu'une partie de la calotte sphérique avec des bords sensiblement droit avec des arrondis, voir par exemple la figure 2 au niveau du haut et du bas.

Les dimensions hors-tout du produit sont : longueur = 240 mm, largeur = 190 mm, hauteur = 115 mm.

La ceinture est composée des deux éléments suivants : un réseau de cavités supports des TUS et une double membrane.

Le réseau de cavités supports des TUS a un entraxe compris entre 38 et 46 mm. Dans l'exemple représenté ci-après, l'entraxe est de 45 mm entre 2 mailles avec une tolérance qui est d'environ ± 0,2 mm.

La dimension du réseau est de 200 mm x 310 mm avec une tolérance d'environ ± 1 mm. Il est possible d'utiliser un mètre ruban pour obtenir ces résultats.

La double membrane est composée d'une première couche support en matière élastique et une deuxième couche support également en matière élastique. Une « membrane » est caractérisée par une épaisseur sensiblement constante.

L'épaisseur de la membrane élastique est de 1,5 mm. Pour cette valeur, une tolérance est d'environ ± 0,2 mm.

La dimension totale avec la double membrane est : 230 mm x 360 mm. Pour ces valeurs, une tolérance est d'environ ± 1 mm. Il est possible d'utiliser un mètre ruban pour obtenir ces résultats.

Les caractéristiques mécaniques de la matière élastique est de l'élastomère silicone sont :
- allongement à rupture (méthode de test : ISO 37) : 500 à 1000 %
- bonne résistance à la rupture,
- silicone grade médical (biocompatible pour le contact dermique)

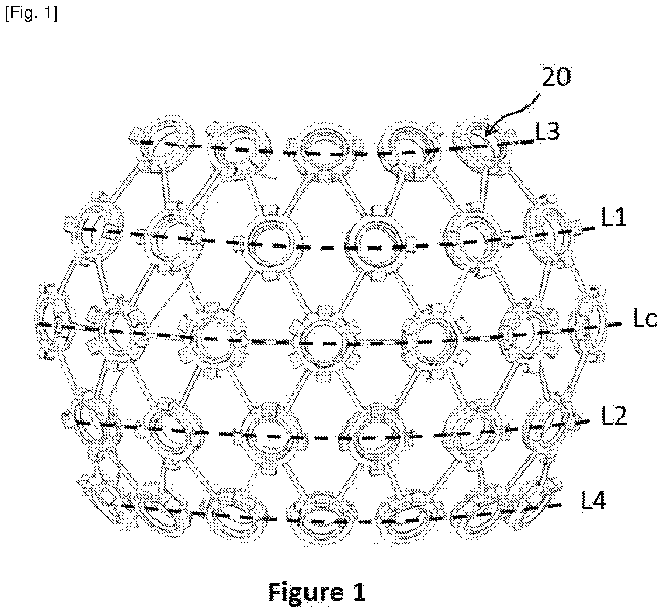

La figure 1 montre un exemple de réalisation comportant le réseau de cavités supports pour trente et un TUS. Chaque TUS est clipsé dans les cavités supports.

Les cavités supports 20 sont disposés de la manière suivante :
- sept cavités supports 20 suivent une ligne centrale Le définissant une ligne de symétrie et séparées chacune par un lien rigide,
- six cavités supports 20 suivent une première ligne L1 parallèle à la ligne centrale Lc, la première ligne L1 est proximale à la ligne centrale Le et située d'un côté, chaque cavité support 20 de la première ligne L1 est positionnée en alternance avec deux cavités supports 20 de la ligne centrale Le et reliée à chacune des deux cavités supports 20 de la ligne centrale Le par un lien rigide,
- six cavités supports 20 suivent une deuxième ligne L2 parallèle à la ligne centrale Lc, la deuxième ligne L2 est proximale à la ligne centrale Le et située de l'autre côté de la ligne centrale Lc, chaque cavité support 20 de la deuxième ligne L2 est positionnée en alternance avec deux cavités supports 20 de la ligne centrale Le et reliée à chacune des deux cavités supports 20 de la ligne centrale Le par un lien rigide,
- cinq cavités supports 20 suivent une troisième ligne L3 parallèle à la ligne centrale, la troisième ligne L3 est distale à la ligne centrale et située du côté de la première ligne L1, chaque cavité support 20 de la troisième ligne L3 est positionnée en alternance avec deux cavités supports 20 de la première ligne L1 et reliée à chacune des deux cavités supports 20 de la première ligne L1 par un lien rigide,
- cinq cavités supports 20 suivent une quatrième ligne L4 parallèle à la ligne centrale Lc, la quatrième ligne L4 est distale à la ligne centrale Le et située du côté de la deuxième ligne L2, chaque cavité support 20 de la quatrième ligne L4 est positionnée en alternance avec deux cavités supports 20 de la deuxième ligne L2 et reliée à chacune des deux cavités supports 20 de la deuxième ligne L2 par un lien rigide.

Dans cet exemple, les liens rigides ont une longueur de 45 mm (distance entre les entraxes des transducteurs).

Dans cet exemple, l'axe longitudinal de chaque lien rigide forme un angle de 60° par rapport à une ligne parallèle à la ligne centrale.

La figure 2 montre les éléments de la figure 1 recouverts par une membrane composée de la première couche support en matière élastique et de la deuxième couche support en matière élastique. Il est montré les cavités supports 20 des 31 TUS.

Cette figure montre le boitier de connexion 21 qui permet de relier tous les TUS à un élément de pilotage. Le boitier de connexion 21 permet de lire les données des TUS ou de transmettre les données à un élément de pilotage déporté.

Dans un autre exemple, des fils électriques reliés à chaque TUS se rejoignent pour aboutir à un câble coaxial qui sort de la ceinture. Ce câble est relié à un boitier électronique inclus ou non (autre version) dans la ceinture textile. Les fils électriques sont situés entre les deux membranes et sont ainsi protégés de l'eau ou de toute agression mécanique.

Dans un autre exemple, le boitier de connexion 21 n'est pas sur la ceinture, mais déporté par rapport à celui-ci.

L'application de la ceinture sur la patiente se fait de la manière suivante : le nombril de la patiente est sensiblement aligné avec la première ligne L1.

Sur la figure 2, il est montré un trou débouchant 22 pour le positionnement du nombril de la patiente.

Le trou débouchant 22 a pour but de faciliter le positionnement par la patiente.

La ceinture de cardiotocographie présente un trou débouchant (absence de matière traversante) permettant à l'utilisateur ou à la patiente d'avoir un repère visuel et/ou un repère tactile constitué par le/son nombril. Ce repérage du nombril est situé entre la première ligne (L1) et la ligne centrale (Lc), il est dans une position centrale de la ceinture de cardiotocographie.

Dans une variante, le trou débouchant 22 est positionné sensiblement au milieu de la première ligne L1.

Ainsi, la position des TUS sur l'abdomen est identique d'un examen à l'autre pour la même patiente grâce à ce repère morphologique.

Sur la figure 3 montre un angle d'environ 30° formé par un triangle rectangle. La base est colinéaire avec l'axe de la ligne centrale, l'hypoténuse correspond au lien rigide et le côté opposé à l'angle de 30° correspond à la perpendiculaire à la base.

La mesure est faite d'un centre de cavité support à un autre centre de cavité support. Dans cet exemple, l'hypoténuse est de 45 mm.

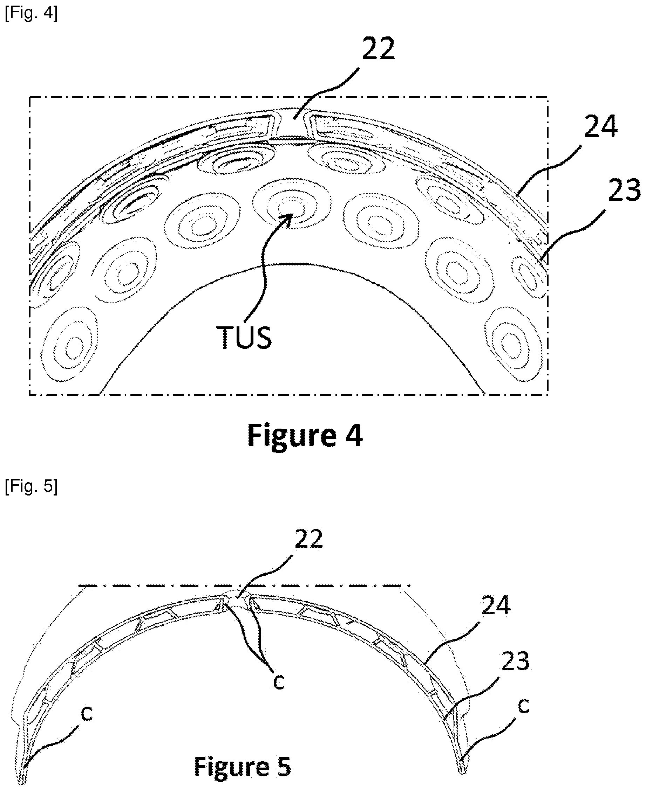

La figure 4 montre une vue en coupe d'une ceinture de cardiotocographie. Il est visible la première couche support 23 et la deuxième couche support 24, le trou de débouchant 22 (trou de repérage pour la patiente) et les TUS (la flèche désigne qu'un seul TUS mais il est visible une pluralité de TUS.

La figure 5 montre une autre vue en coupe d'une ceinture de cardiotocographie montrant le collage, noté c, de la première couche support 23 et de la deuxième couche support 24.

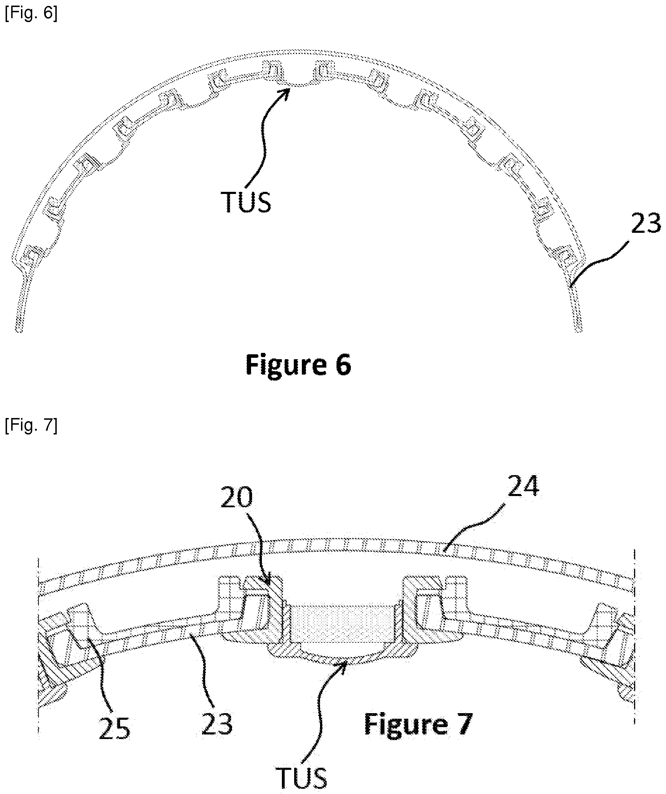

La figure 6 montre une autre vue en coupe selon la ligne LC et il est visible des TUS en coupe (représentation partielle des TUS), la première couche support 23 et de la deuxième couche support 24.

La figure 7 montre une vue de détail de la coupe précédente. Il est également visible des TUS en coupe, la première couche support 23 et de la deuxième couche support 24.

Il est représenté et référencé des liens entre les cavités support 25.

Sur cette figure, les TUS est matérialisé par un rectangle qui comporte un réceptacle visible en coupe.

Pour rappel, l'étanchéité de la partie technique est assurée par le clippage des TUS sur les cavités supports 20 et par le collage des deux membranes entre elles.

Selon un exemple de réalisation, le processus d'assemblage permettant l'étanchéité est réalisé par le vissage avec joint, le collage avec colle ou ruban adhésif, le clippage, le surmoulage de la membrane inférieure sur le réseau de cavité support, la soudure par Ultrason des TUS sur le réseau de cavité support. L'étanchéité est réalisée selon ce mode de réalisation. Toutefois, toute autre manière d'assemblage des TUS dans les cavités supports permettant l'étanchéité est envisagé.

### LISTE DES SIGNES DE RÉFÉRENCE

**[Table 1]**

| Références | Désignations |
|---|---|
| 20 | cavité support |
| 21 | boîtier de connexion |
| 22 | trou de repérage |
| 23 | première couche support |
| 24 | deuxième couche support |
| 25 | liens entre les cavités support |
| TUS | transducteurs ultrasonores |
| Lc | ligne centrale |
| L1 | première ligne |
| L2 | deuxième ligne |
| L3 | troisième ligne |
| L4 | quatrième ligne |

## Revendications

1. Ceinture de cardiotocographie, **caractérisée en ce qu'**elle a une forme d'une calotte sphérique et comporte au moins dix-neuf transducteurs ultrasonores, appelé TUS, positionnés sur une première couche support (23) en matière élastique, chaque TUS étant positionné dans une cavité support (20), ladite ceinture comporte une deuxième couche support (24) également en matière élastique qui encapsule lesdits TUS logés dans les cavités supports (20) avec la première couche support (23), l'assemblage est réalisé par collage de la première couche support (23) avec la deuxième couche support (24).

2. Ceinture selon la revendication 1, dans laquelle lesdites cavités supports (20) étant disposées de la manière suivante dont au moins :
- cinq cavités supports (20) suivent une ligne centrale (Lc) définissant une ligne de symétrie et séparées chacune par un lien rigide,
- quatre cavités supports (20) suivent une première ligne (L1) parallèle à la ligne centrale (Lc), la première ligne (L1) est proximale à la ligne centrale (Lc) et située d'un côté, chaque cavité support (20) de la première ligne (L1) est positionnée en alternance avec deux cavités supports (20) de la ligne centrale (Lc) et reliée à chacune des deux cavités supports (20) de la ligne centrale (Lc) par un lien rigide,
- quatre cavités supports (20) suivent une deuxième ligne (L2) parallèle à la ligne centrale (Lc), la deuxième ligne (L2) est proximale à la ligne centrale (Lc) et située de l'autre côté de la ligne centrale (Lc), chaque cavité support (20) de la deuxième ligne (L2) est positionnée en alternance avec deux cavités supports (20) de la ligne centrale (Lc) et reliée à chacune des deux cavités supports (20) de la ligne centrale (Lc) par un lien rigide,
- trois cavités supports (20) suivent une troisième ligne (L3) parallèle à la ligne centrale (Lc), la troisième ligne (L3) est distale à la ligne centrale (Lc) et située du côté de la première ligne (L1), chaque cavité support (20) de la troisième ligne (L3) est positionnée en alternance avec deux cavités supports (20) de la première ligne (L1) et reliée à chacune des deux cavités supports (20) de la première ligne (L1) par un lien rigide,
- trois cavités supports (20) suivent une quatrième ligne (L4) parallèle à la ligne centrale (Lc), la quatrième ligne (L4) est distale à la ligne centrale (Lc) et située du côté de la deuxième ligne (L2), chaque cavité support (20) de la quatrième ligne (L4) est positionnée en alternance avec deux cavités supports (20) de la deuxième ligne (L2) et reliée à chacune des deux cavités supports (20) de la deuxième ligne (L2) par un lien rigide.

3. Ceinture selon la revendication 1, dans laquelle l'épaisseur de la première couche ou de la deuxième couche support (24) est comprise entre 1 et 2 mm, de préférence compris entre 1,3 et 1,7 mm.

4. Ceinture selon la revendication 2, dans laquelle la distance proximale entre la première ligne (L1) et la ligne centrale (Lc) ou la deuxième ligne (L2) et la ligne centrale (Lc) est compris entre 32 et 40 mm.

5. Ceinture selon la revendication 2, dans laquelle la distance distale entre la troisième ligne (L3) et la ligne centrale (Lc) ou la quatrième ligne (L4) et la ligne centrale (Lc) est compris entre 64 et 80 mm.

6. Ceinture selon la revendication 2, dans laquelle le lien rigide entre la ligne centrale (Lc) et la première ligne (L1) ou la ligne centrale (Lc) et la deuxième ligne (L2) forme un angle compris entre 50 et 70°par rapport à la ligne centrale (Lc).

7. Ceinture selon la revendication 2, dans laquelle le lien rigide entre la première ligne (L1) et la troisième ligne (L3) ou la deuxième ligne (L2) et la quatrième ligne (L4) forme un angle compris entre 50 et 70°par rapport à la ligne centrale (Lc).

8. Ceinture selon la revendication 2, dans laquelle chaque lien rigide a une longueur entre 38 et 46 mm.

9. Ceinture selon la revendication 1, dans laquelle la matière élastique est du silicone.

10. Ceinture selon la revendication 2, dans laquelle un trou débouchant (22) est situé entre la première ligne (L1) et la ligne centrale (Lc), ledit trou débouchant (22) est sensiblement en position centrale de ladite ceinture.

11. Ceinture selon la revendication 1, dans laquelle un trou débouchant (22) est sensiblement en position centrale de ladite ceinture.

## Patentansprüche

1. Kardiotokographiegürtel, **dadurch gekennzeichnet, dass** er eine Form einer Kugelkalotte hat und mindestens neunzehn Ultraschallwandler, bezeichnet als TUS, aufweist, die auf einer ersten Trägerschicht (23) aus elastischem Material positioniert sind, wobei jeder TUS in einem Trägerhohlraum (20) positioniert ist, der Gürtel eine zweite Trägerschicht (24) ebenfalls aus elastischem Material aufweist, die die in den Trägerhohlräumen (20) untergebrachten TUS mit der ersten Trägerschicht (23) einkapselt, wobei die Verbindung durch Verkleben der ersten Trägerschicht (23) mit der zweiten Trägerschicht (24) erfolgt.

2. Gürtel nach Anspruch 1, wobei die Trägerhohlräume (20) wie folgt angeordnet sind, wobei mindestens:
- fünf Trägerhohlräume (20) einer Mittellinie (Lc) folgen, die eine Symmetrielinie definiert, und jeweils durch eine starre Verbindung getrennt sind,
- vier Trägerhohlräume (20) einer ersten Linie (L1) parallel zur Mittellinie (Lc) folgen, wobei die erste Linie (L1) proximal zur Mittellinie (Lc) ist und sich auf einer Seite befindet, wobei jeder Trägerhohlraum (20) der ersten Linie (L1) abwechselnd mit zwei Trägerhohlräumen (20) der Mittellinie (Lc) positioniert ist und mit jedem der beiden Trägerhohlräume (20) der Mittellinie (Lc) durch eine starre Verbindung verbunden ist,
- vier Trägerhohlräume (20) einer zweiten Linie (L2) parallel zur Mittellinie (Lc) folgen, wobei die zweite Linie (L2) proximal zur Mittellinie (Lc) ist und sich auf der anderen Seite der Mittellinie (Lc) befindet, wobei jeder Trägerhohlraum (20) der zweiten Linie (L2) abwechselnd mit zwei Trägerhohlräumen (20) der Mittellinie (Lc) positioniert ist und mit jedem der beiden Trägerhohlräume (20) der Mittellinie (Lc) durch eine starre Verbindung verbunden ist,
- drei Trägerhohlräume (20) einer dritten Linie (L3) parallel zur Mittellinie (Lc) folgen, wobei die dritte Linie (L3) distal zur Mittellinie (Lc) ist und sich auf der Seite der ersten Linie (L1) befindet, wobei jeder Trägerhohlraum (20) der dritten Linie (L3) abwechselnd mit zwei Trägerhohlräumen (20) der ersten Linie (L1) positioniert ist und mit jedem der beiden Trägerhohlräume (20) der ersten Linie (L1) durch eine starre Verbindung verbunden ist,
- drei Trägerhohlräume (20) einer vierten Linie (L4) parallel zur Mittellinie (Lc) folgen, wobei die vierte Linie (L4) distal zur Mittellinie (Lc) ist und sich auf der Seite der zweiten Linie (L2) befindet, wobei jeder Trägerhohlraum (20) der vierten Linie (L4) abwechselnd mit zwei Trägerhohlräumen (20) der zweiten Linie (L2) positioniert ist und mit jedem der beiden Trägerhohlräume (20) der zweiten Linie (L2) durch eine starre Verbindung verbunden ist.

3. Gürtel nach Anspruch 1, wobei die Dicke der ersten Schicht oder zweiten Trägerschicht (24) zwischen 1 und 2 mm, vorzugsweise zwischen 1,3 und 1,7 mm, beträgt.

4. Gürtel nach Anspruch 2, wobei der proximale Abstand zwischen der ersten Linie (L1) und der Mittellinie (Lc) oder der zweiten Linie (L2) und der Mittellinie (Lc) zwischen 32 und 40 mm beträgt.

5. Gürtel nach Anspruch 2, wobei der distale Abstand zwischen der dritten Linie (L3) und der Mittellinie (Lc) oder der vierten Linie (L4) und der Mittellinie (Lc) zwischen 64 und 80 mm beträgt.

6. Gürtel nach Anspruch 2, wobei die starre Verbindung zwischen der Mittellinie (Lc) und der ersten Linie (L1) oder der Mittellinie (Lc) und der zweiten Linie (L2) einen Winkel zwischen 50 und 70° im Verhältnis zur Mittellinie (Lc) bildet.

7. Gürtel nach Anspruch 2, wobei die starre Verbindung zwischen der ersten Linie (L1) und der dritten Linie (L3) oder der zweiten Linie (L2) und der vierten Linie (L4) einen Winkel zwischen 50 und 70° im Verhältnis zur Mittellinie (Lc) bildet.

8. Gürtel nach Anspruch 2, wobei jede starre Verbindung eine Länge zwischen 38 und 46 mm aufweist.

9. Gürtel nach Anspruch 1, wobei das elastische Material Silikon ist.

10. Gürtel nach Anspruch 2, wobei sich ein Durchgangsloch (22) zwischen der ersten Linie (L1) und der Mittellinie (Lc) befindet, wobei das Durchgangsloch (22) etwa in der Mittelposition des Gürtels befindet.

11. Gürtel nach Anspruch 1, wobei sich ein Durchgangsloch (22) etwa in der Mittelposition des Gürtels befindet.

## Claims

1. A cardiotocography girdle, **characterised in that** it is in the form of a spherical dome and includes at least nineteen ultrasound transducers, referred to as USTs, which are positioned on a first support layer (23) made of an elastic material, each UST being positioned in a support cavity (20), said girdle comprises a second support layer (24), likewise made of an elastic material and which encapsulates said USTs housed in the support cavities (20) with the first support layer (23), the assembly being performed by adhesively bonding the first support layer (23) to the second support layer (24).

2. The girdle according to claim 1, wherein said support cavities (20) are arranged in the following manner, in which at least:
- five support cavities (20) follow a central line (Lc) defining a line of symmetry each separated by a rigid link,
- four support cavities (20) follow a first line (L1) parallel to the central line (Lc), the first line (L1) is proximal to the central line (Lc) and situated on one side, each support cavity (20) of the first line (L1) is alternately positioned with two support cavities (20) of the central line (Lc) and connected to each of the two support cavities (20) of the central line (Lc) by a rigid link,
- four support cavities (20) follow a second line (L2) parallel to the central line (Lc), the second line (L2) is proximal to the central line (Lc) and situated on the other side of the central line (Lc), each support cavity (20) of the second line (L2) is alternately positioned with two support cavities (20) of the central line (Lc) and connected to each of the two support cavities (20) of the central line (Lc) by a rigid link,
- three support cavities (20) follow a third line (L3) parallel to the central line (Lc), the third line (L3) is distal from the central line (Lc) and situated on the side of the first line (L1), each support cavity (20) of the third line (L3) is alternately positioned with two support cavities (20) of the first line (L1) and connected to each of the two support cavities (20) of the first line (L1) by a rigid link,
- three support cavities (20) follow a fourth line (L4) parallel to the central line (Lc), the fourth line (L4) is distal from the central line (Lc) and situated on the side of the second line (L2), each support cavity (20) of the fourth line (L4) is alternately positioned with two support cavities (20) of the second line (L2) and connected to each of the two support cavities (20) of the second line (L2) by a rigid link,

3. The girdle according to claim 1, wherein the thickness of the first layer or of the second support layer (24) is between 1 and 2 mm, preferably between 1.3 and 1.7 mm.

4. The girdle according to claim 2, wherein the proximal distance between the first line (L1) and the central line (Lc) or the second line (L2) and the central line (Lc) is between 32 and 40 mm.

5. The girdle according to claim 2, wherein the distal distance between the third line (L3) and the central line (Lc) or the fourth line (L4) and the central line (Lc) is between 64 and 80mm.

6. The girdle according to claim 2, wherein the rigid link between the central line (Lc) and the first line (L1) or the central line (Lc) and the second line (L2) forms an angle of between 50 and 70° with respect to the central line (Lc).

7. The girdle according to claim 2, wherein the rigid link between the first line (L1) and the third line (L3), or between the second line (L2) and the fourth line (L4), forms an angle between 50 and 70° with respect to the central line (Lc).

8. The girdle according to claim 2, wherein each rigid link has a length between 38 and 46 mm.

9. The girdle according to claim 1, wherein the elastic material is silicone.

10. The girdle according to claim 2, wherein a through-hole (22) is located between the first line (L1) and the central line (Lc), said through-hole (22) being substantially in a central position of said girdle.

11. The girdle according to claim 1, wherein a through-hole (22) is substantially in a central position of said girdle.
